# EUROPÄISCHE PATENTANMELDUNG

(11) **EP 3 017 712 A1**
(43) Veröffentlichungstag der Anmeldung: **11.05.2016**
(21) Anmeldenummer: 15191529.5
(22) Anmeldetag: 26.10.2015
(51) Int. Cl.: A43B 7/20, A61F 5/01

(54) **MEDIZINISCHER STABILSCHUH ZUR SPRUNGGELENKSTABILISIERUNG MIT INTEGRIERTEM STÜTZSYSTEM UND REGULIERBAREM GELENK**

(30) Priorität: 06.11.2014 CH 17182014
(71) Anmelder: Künzli SwissSchuh AG, 5210 Windisch (CH)
(72) Erfinder: ARTMANN, Barbara, 5408 Ennetbaden (CH); BERISHA, Skender, 5033 Buchs (CH); BÜCHLI, Katharina, 5076 Bözen (CH); WÜRSCH, Florian, 4053 Basel (CH)
(74) Vertreter: Bremi, Tobias Hans

(57) **Zusammenfassung**

Schuhwerk, oder bewegliche Fussstütze in Form einer Bandage, mit einer den Fuss wenigstens im Fersen- und hinteren Sohlenbereich, im Knöchelbereich und oberhalb des Knöchelbereichs umgreifenden Schaftstruktur (12) und einem Exoskelett (13), das an und/oder in der Schaftstruktur (12) befestigt ist, wobei das Exoskelett (13) ein in einem Sohlenbereich (11) der Schaftstruktur (12) befestigtes steifes Schalenelement (14,15) aufweist, das die Unterseite des Fusses wenigstens teilweise untergreift und das sich beidseits des Fusses in Form von Erweiterungen (14a, 15a) bis auf oberhalb der Höhe des Drehpunktes des Fussgelenks erstreckt, sowie ein steifes Stützelement (16), welches eine den Unterschenkel wenigstens teilweise oberhalb des Fussgelenks umlaufende Manschette (17) aufweist, sowie zwei seitliche, nach unten gerichtete Versteifungen (18, 19), welche sich beidseits des Fusses bis auf unterhalb der Höhe des Drehpunktes des Fussgelenks erstrecken, wobei Erweiterungen (14a, 15a) und Versteifungen (18,19) beidseits des Fusses über je ein fussaussenseitiges und ein fussinnenseitiges Gelenk (20,21) verschwenkbar verbunden sind, und wobei der maximale Öffnungswinkel des Gelenks (20, 21) bezüglich Plantarflexion begrenzt ist durch wenigstens ein dehnungsarmes Band (25), welches wenigstens eine Erweiterung (14a, 15a) fusspitzenseitig mit einem schienbeinseitigen oder seitlichen Bereich der Manschette (17) einstellbar verbindet.

## Beschreibung

### TECHNISCHES GEBIET

Die vorliegende Erfindung betrifft einen Schuh, insbesondere einen orthopädischen Spezialschuh mit integrierter Sprunggelenksstabilisierung, mit seitlichen Versteifungen, die mit je einem Gelenk versehen einen gewissen Schwenkbereich des Sprunggelenks zulassen, und mit Hilfe von vor- und nachgelagerten Bändern einstellbar sind, und welche den Bewegungsbereich des Gelenks limitieren, ggf. unterstützt vom einem eingebauten und modifizierbarem Anschlag beim Gelenk, um die Therapie von Sprunggelenksverletzungen gezielt je nach Verletzung und Verletzungsgrad anpassen zu können.

### STAND DER TECHNIK

Im Gebiet der orthopädischen Hilfsmittel zur Sprunggelenkstabilisierung existieren verschiedene Hilfsmittelarten wie z.B. Bandagen aus Textilien, teilweise verbunden mit Kunststoffelementen, Orthesen aus Hartplastik, Schienen, Gipsverbände und orthopädische Stabilschuhe. Die Hilfsmittel werden als medizinische Therapie bei verschiedenen Verletzungen am Sprunggelenk zur Ruhigstellung bei der Heilung eingesetzt, mit einem Trend zur frühfunktionellen Belastung anstatt totaler Fixierung der Gelenke wie dies bei einem Gipsverband der Fall ist. Dabei sollen Inversions- und Eversionsbewegungen des Fusses möglichst gering ausfallen, während Plantarflexion und Dorsalextension im oberen Sprunggelenk je nach Verletzung innerhalb eines gewissen Bewegungsausmasses erwünscht sind.

Die CH 662483 beschreibt einen Stabilschuh mit seitlichen, in am Schuh vorgesehene Taschen eingeschobene stabförmige Stabilisatoren, welche in den Schaft integriert für die nötige seitliche Stabilität sorgen, und auch die Plantarflexion und Dorsalextension einschränken, dies jedoch gekoppelt mit der seitlichen Stabilität, daher sind die einzelnen Bewegungen nicht unabhängig regulierbar. Zudem ist der Einstieg in einer neutralen und verletzungsschonenden Position durch die durchgehende Schnürung umständlich.

Die US 4719926 beschreibt eine Bandage, welche Plantarflexion und Dorsalextension durch eine seitlich am Fuss befestigte Gelenks-Schiene führt und limitiert. Das Bewegungsausmass ist dabei durch die Länge eines bogenförmigen Langloches in der Gelenks-Schiene, in welches ein Stift eines den Fuss unten umgreifenden Elements verschieblich eingreift, vorgegeben und nicht regulierbar.

In der US 6350246 und der US 5865778 sind rigide Schienen beschrieben, welche durch eine Gelenksachse Plantarflexion und Dorsalextension zulassen. Durch eine direkt bei der Gelenkachse integrierte Schiene mit Stop-Bolzen wird das Bewegungsausmass über die und bei der Gelenkachse limitiert. Ein ähnliches Prinzip wird in der WO 02085148 beschrieben, in welcher der Schuh ebenfalls eine rigide Struktur mit Dreh-Gelenk aufweist. Das Dreh-Gelenk wird bei dieser Offenbarung durch eine etwas ausserhalb des Gelenks liegende Schiene mit Stop-Bolzen limitiert. Bei allen drei Varianten sind dabei die Kräfte, welche auf den Stop-Bolzen wirken, sehr hoch und die einzelne Regulierung von Plantarflexion und Dorsalextension, ist wenn überhaupt nur mit aufwändigen Umstellungen verbunden vorgesehen.

Die DE 10 2008 013 382 betrifft eine Stützschalenanordnung, die ebenfalls mit einem Drehgelenk versehen ist, wobei die Gelenksachse der Fussgelenkachse entspricht. Einstellbar ist das Gelenk durch eine Schiebeinrichtung auf einem Fersenteil, welche ebenso gelenkig mit der Stützbasis verbunden ist. Die Einstellung erfolgt dabei mittels veränderbaren Endanschlags, welcher auf einer Führungsschiene verschoben werden kann. Die einzelnen Bewegungen sind so regulierbar. Der Nachteil dieser Konstruktion liegt im grossen Volumen, welche diese einnimmt und dadurch im Alltag hinderlich ist.

### DARSTELLUNG DER ERFINDUNG

Die Aufgabe der vorliegenden Erfindung liegt darin, ein orthopädisches Hilfsmittel bei Verletzungen im oberen Sprunggelenk in Form eines Stabilschuhs bereitzustellen, der in Funktion einer Orthese eine Ruhigstellung für die Verletzung genau im jeweils medizinisch vorgegebenen Mass einfach und stabil erlaubt und zudem (als Schuh) frühfunktionelle Therapie durch eine gewisse Belastung des verletzten Fusses von Beginn der Therapie an ermöglicht, einen physiologischen Gang und verletzungsschonendes An- und Ausziehen vorsieht sowie vorzugsweise das Abbauen im Therapieverlauf überflüssig werdender Komponenten zulässt.

Die Distorsion des oberen Sprunggelenks ist die häufigste Ursache von Fussverletzungen.

Der zugrunde liegende Verletzungsmechanismus umfasst eine Kombination von Plantarflexion (Streckung des Sprunggelenks), Adduktion (Heranführung zum Körper) und Inversion (Umkehrung) des Fusses, auch Supinationsbewegung genannt, welche bei unkontrolliertem Ausmass zu einem Supinationstrauma (=Distorsion) führen kann. In der Therapie solcher Verletzungen müssen die verletzten Strukturen gezielt innerhalb gewisser Belastungsgrenzen stabilisiert werden und gleichzeitig bei frühfunktioneller Belastung die Bewegungsapparatur aktiv gehalten werden, um z.B. einen unerwünschten Muskelschwund zu vermeiden. Der hierzu vorzusehende Stabilschuh soll daher nach anerkannten medizinischen Vorgaben anpassbar sein, indem die seitliche Stabilität mit Inversion und Eversion, wie auch die Plantarflexion und Dorsalextension regulierbar und an den Heilungsverlauf anpassbar sind. Das medizinisch definierte Mass an Bewegungsfreiheit und die frühfunktionelle Belastung sollen eine steuerbare Therapie und damit sowohl eine schnellere Heilung wie auch mehr Sicherheit für Arzt und Patient (Compliance) bewirken.

Diese Aufgabe wird durch einen Spezialschuh mit hohem Schaft in Kombination mit einem steifen Exoskelett gelöst, welches die wichtige seitliche Stabilität gewährleistet. Ein solcher Schuh wird durch die Merkmale von Anspruch 1, und in bevorzugten Varianten durch die Merkmale der anhängigen Ansprüche, zur Verfügung gestellt.

Im Therapieverlauf kann die seitliche Stabilität durch das Weglassen eines vorgelagerten Bandes sowie das Demontieren des Stützelements verringert werden. Ein Drehgelenk etwa auf der Höhe der Bewegungsachse des oberen Sprunggelenks lässt eine physiologische Plantarflexion und Dorsalextension zu, wobei sich die Bewegungen abhängig von der Verletzung einzeln primär mit Hilfe von vor- und nachgelagerten Bändern einschränken lassen oder auch nicht und sekundär ggf. im Drehgelenk Endanschläge für verschiedene Bewegungsausmasse realisierbar sind oder auch nicht.

Konkret betrifft die vorliegende Erfindung damit ein Schuhwerk, oder eine bewegliche Fussstütze in Form einer Bandage, mit einer den Fuss wenigstens im Fersen- und hinteren Sohlenbereich, im Knöchelbereich und oberhalb des Knöchelbereichs umgreifenden flexiblen Schaftstruktur und einem wenigstens teilweise ausserhalb davon angeordneten Exoskelett, das an und/oder in der Schaftstruktur befestigt ist.

Dabei umfasst das Exoskelett ein Schalenelement und ein Stützelement, beide sind als steife Elemente ausgebildet.

Das Schalenelement ist in einem Sohlenbereich der Schaftstruktur befestigt und untergreift die Unterseite des Fusses wenigstens teilweise. Es erstreckt sich beidseits des Fusses in Form von Erweiterungen bis auf oberhalb der Höhe des Drehpunktes des Fussgelenks.

Das steife Stützelement weist eine den Unterschenkel wenigstens teilweise oberhalb des Fussgelenks umlaufende Manschette auf, sowie zwei seitliche, nach unten gerichtete Versteifungen, welche sich beidseits des Fusses bis auf unterhalb der Höhe des Drehpunktes des Fussgelenks erstrecken.

Dabei sind die Erweiterungen und Versteifungen beidseits des Fusses über je ein fussaussenseitiges und ein fussinnenseitiges Gelenk verschwenkbar verbunden.

Der maximale Öffnungswinkel des Gelenks bezüglich Plantarflexion ist also primär begrenzt durch wenigstens ein dehnungsarmes Band, welches wenigstens eine Erweiterung fusspitzenseitig davon mit einem schienbeinseitigen oder seitlichen Bereich der Manschette einstellbar verbindet, und dabei vorzugsweise quer über den Talus verläuft. Das fussaussenseitige und/oder das fussinnenseitige Gelenk können dabei zusätzlich mit Endanschlägen für die verschiedene Bewegungsausmasse der Plantarflexion und Dorsalextension versehen sein. Das hier und in der Folge beschriebene Gelenk ist dabei für sich betrachtet eine Innovation im vorliegenden Zusammenhang aber auch im Zusammenhang mit einer Fussstütze, die nicht notwendigerweise die oben beschriebenen Merkmale aufweisen muss. Auch unabhängig von den Merkmalen von Anspruch 1 ist also ein Gelenk mit den Merkmalen, wie sie in der Folge beschrieben werden, als ein weiterer Gegenstand der vorliegenden Anmeldung zu betrachten.

Konstruktiv können solche Endanschläge für die verschiedene Bewegungsausmasse der Plantarflexion und Dorsalextension vorzugsweise umgesetzt sein durch mindestens zwei gegenüberliegende, definierte, bogenförmige Öffnungen am Drehgelenk, welche als Führungsschiene für einen entsprechenden fix montierten Gelenkdeckel mit mindestens zwei, um die Kräfte zu verteilen, bezüglich der Gelenksachse gegenüberliegenden integrierten Stiften dienen. Die Stifte verhindern dadurch bei einer Drehbewegung im Drehgelenk durch Anschlag am Ende der bogenförmigen Öffnungen eine weitere Drehung und ermöglichen so je nach Durchmesser der Stifte und Länge der Öffnungen verschiedene Öffnungswinkel. Dieser zusätzliche Gelenksanschlag unterbindet jedoch bevorzugt nicht das physiologischen Gehen, sondern erst Bewegungen ausserhalb der medizinischen Vorgaben, wie zum Beispiel Auslenkungen beim ungeplanten Stolpern.

Um generell einen zu abrupten Endanschlag zu verhindern wird das Bewegungsausmass vorzugsweise jeweils schon zuvor durch vor- und nachgelagerte Bänder sanfter gebremst und somit möglichst Bewegungen, welche zu einem Endanschlag führen können, präventiv verhindert, indem der Verletzte den Bremseffekt realisiert und entsprechend mehr Zeit hat zu reagieren.

Eine weitere bevorzugte Ausführungsform eines derartigen Gelenks, umfasst zwei Bereiche, einen äusseren Gelenkbereich vorzugsweise mit einer Gewindehülse, welche durch eine äussere Gelenkfläche mit einem Gelenkdeckel verbunden ist und beispielsweise bei der Montage mit dem Gelenkdeckel verpresst wird. Der innere Gelenkbereich bildet sich aus einem Verdrehelement, das eine innere Gelenkfläche mit der Gegenplatte verbindet. Die Verbindung zwischen Verdrehelement und Gegenplatte kann dabei eine Steckverbindung sein. Vorzugsweise ist der äussere Gelenkbereich mit dem innerem Gelenkbereich verbunden, beispielsweise über ein Gewinde.

Die innere und äussere Gelenkfläche können dabei vorteilhafter Weise mit jeweils vier bogenförmigen Ausnehmungen oder Öffnungen versehen sein, wobei die Öffnungen jeweils um 90° um die Achse des Gelenks verschoben sind, so dass sich zwei Mal paarweise zwei gleiche Öffnungen gegenüberliegen. Auf der Gegenplatte sind dann bevorzugter Massen zwei gegenüberliegende Stifte angeordnet, welche vom Fuss weg zeigen und in die bogenförmigen Öffnungen auf der inneren und äusseren Gelenkfläche greifen. Auf der äusseren Gelenkfläche besitzen die bogenförmigen Öffnungen alle dieselbe Dimension und entsprechen in der Grösse den zwei gegenüberliegenden Stiften auf der Gegenplatte. Die Öffnungen auf der inneren Gelenkfläche verfügen hingegen über eine grössere Länge (in Umfangsrichtung) als die Stifte auf der Gegenplatte. Die Dimensionierung ist auch umgekehrt möglich, d.h. das in der äusseren Gelenkfläche paarweise Öffnungen mit unterschiedlicher umfangmäßiger Länge ausgebildet sind, und auf der inneren Gelenkfläche alle vier Öffnungen in Umfangsrichtung gleich lang sind. Greifen die Stifte in die längeren bogenförmigen Öffnungen ist damit die Beweglichkeit um das Gelenk grösser. Diese vorgeschlagene Konstruktion weist den konstruktiv einfach realisierten Vorteil auf, dass die Gegenplatte um 90° um die Gelenksachse gedreht werden kann, um den zugänglichen Winkelbereich des Gelenks einzustellen. Je nachdem wie die Gegenplatte in ihrer Rotationsposition um die Gelenkachse positioniert wird, ist so eine Anpassung des Öffnungswinkels möglich und verschiedene Endanschläge für den Bewegungsspielraum sind je nach Therapiephase realisierbar.

Um das Anziehen der Schuhe zu erleichtern, lässt sich gemäss einer weiteren bevorzugten Ausführungsform das ganze Stützelement abklappen. Durch Drehung am Gelenkdeckel und die fixe Verbindung mit der Gewindehülse kann diese beispielsweise aus dem Verdrehelement gedreht werden und schiebt so den äusseren und inneren Gelenkbereich auseinander. Dadurch greifen die Stifte nicht mehr in die Ausnehmungen der äusseren Gelenkfläche und der gesamte äussere Gelenkbereich ist entkoppelt. Das Gelenk lässt sich frei drehen und das Stützelement kann bei geöffneten Bändern nach hinten abgeklappt werden, um den Einstieg zu erleichtern.

Eine weitere bevorzugte Ausführungsform ist dadurch gekennzeichnet, dass das Band an der fussaussenseitigen Erweiterung im Bereich deren der Fussspitze zugewandten Kante befestigt ist und über den Talus laufend am fussinnenseitigen Bereich der Manschette einstellbar festgelegt werden kann, vorzugweise über eine Klettverbindung, die beispielsweise auf der Aussenseite der Manschette und auf der Innenseite des Bandes vorgesehen ist.

Eine weitere bevorzugte Ausführungsform ist dadurch gekennzeichnet, dass zwischen Sohlenbereich und fersenseitiger Manschette ein dehnungsarmes einstellbares zweites Band angeordnet ist, vorzugsweise indem es im Sohlenbereich befestigt, durch einen horizontalen Schlitz in der Manschette geführt und nach unten umgelegt ist und variabel festgelegt werden kann, vorzugsweise über einen Druckverschluss oder eine Klettverbindung. So kann die Beweglichkeit in der Dorsalflexion gezielt und einstellbar beschränkt werden. Weiterhin vorzugsweise handelt es sich um ein Schuhwerk, wobei das Schuhwerk vorzugsweise im den Knöchel umgreifenden Bereich sowie über wenigstens 50 %, insbesondere bevorzugt wenigstens 70 % der Ristlänge öffenbar ist.

Im den Knöchel umgreifenden Bereich kann eine zentral geteilte Zunge angeordnet sein, welche über eine Schnürung verschlossen werden kann.

Im Übergangsbereich zwischen dem knöchelumgreifenden Bereich und dem Ristbereich kann eine dahinterliegende Zwischenlasche angeordnet sein.

Im Ristbereich kann die Einstiegsöffnung für den Fuss über wenigstens eine Klapplasche verschliessbar ausgestaltet sein, vorzugsweise über zwei von der Fussinnenseite respektive der Fussaussenseite zur Mitte zum Rist und wenigstens Bereichsweise übereinandergelegt verschliessbare Klapplaschen.

Die fussaussenseitige Klapplasche verfügt vorzugsweise über ein elastisches Band, welches insbesondere bevorzugt auf der Fussinnenseite, vorzugsweise an der inneren Erweiterung, festgelegt oder wieder nach aussen umgelegt und nach aussen festgelegt werden kann.

Das Schalenelement kann beispielsweise einstückig U-förmig ausgebildet sein oder zwei getrennte L-förmige Elemente aufweisen, ein äusseres und ein inneres, wobei vorzugsweise die äussere Erweiterung sich weiter zur Fussspitze erstreckt als die innere. Die Gelenke können bevorzugter Massen lösbar ausgestaltet sein, sodass das Stützelement im fortgeschrittenen Therapiestadium entfernt werden kann, wobei beispielsweise die Gelenke über eine innere, dem Fuss zugewandte Gelenkplatte verfügen, welche eine nach aussen gerichtete, mit einem Innengewinde versehene hohlzylindrische Erweiterung aufweisen, deren Aussenfläche vorzugsweise die Drehachse bildet, und das Gelenk durch eine von aussen auf einen Gelenkdeckel eingeschraubte Kopfschraube mit in das Innengewinde der hohlzylindrischen Erweiterung eingreifendem Aussengewinde zusammengehalten ist.

Vorzugsweise können zwischen aneinander gleitenden Elementen des jeweiligen Gelenks Gleitelemente, insbesondere vorzugsweise PTFE-Scheiben, angeordnet sein.

Eine weitere bevorzugte Ausführungsform ist dadurch gekennzeichnet, dass die Manschette als zur Fussspitze eine Einstiegsausnehmung aufweisende Ringmanschette ausgebildet ist, deren Einstiegsausnehmung vorzugsweise über ein bandförmiges, vorzugsweise dehnungsarmes Verschlusselement geschlossen werden kann, insbesondere bevorzugt über eine Umlenkrolle auf sich selber zurückgelegt über einen Klettverschluss.

### Exoskelett

Das drucksteife Exoskelett umfasst ein Stützelement und ein Schalenelement, bevorzugt in zwei Teilen, verbunden über ein Drehgelenk. Das Schalenelement ist auf der Fussaussenseite und Fussinnenseite zwischen Laufsohle und Schaft fest montiert und bildet das Fundament. Bei einem Supinationstrauma sind meist die fibularen Seitenbänder betroffen, insbesondere das Ligamentum talofibulare anterius, da sich das Band in Längsrichtung des Schuhs vor dem Knöchel befindet. Das äussere Schalenelement ist daher im Vergleich zum inneren Schalenelement bevorzugt weiter nach vorne Richtung kleine Zehe gezogen um den Fuss beim Abrollvorgang länger zu führen und zusätzlich lateral zu schützen.

Das druckstabile und zugstabile Stützelement umfasst ein zumindest teilweise den Unterschenkel umgreifende Manschette, sowie auf der Fussinnenseite und der Fussaussenseite längsförmige Versteifungen als Stützen zur seitlichen Sprunggelenkstabilisierung, um die für einen verletzten Fuss ungünstige Inversion und Eversion auf ein nicht gefährdendes Minimum zu reduzieren. In einer bevorzugten Ausführung des Manschettenelements ist der obere Rand leicht konvex geformt und im Fersenbereich unter dem Achillessehnenansatz etwas tiefer gezogen, um Druck auf den Unterschenkel und Achillessehnenansatz zu vermeiden. Zudem empfiehlt es sich, eine nicht zu schmale Manschette einzusetzen, besonders im vorderen Abschlussbereich, um eine punktuelle Druckausübung durch die Manschette zu vermeiden. Bevorzugt ist zudem vorgesehen, dass das Manschettenelement mit einem Verschlusselement den gesamten Unterschenkel umgreift und dadurch die Lastenübertragung grossflächig erfolgt.

Die Schalenelemente und das Stützelement sind mit einem Drehgelenk verbunden, welches sich möglichst auf Höhe der natürlichen Bewegungsachse des oberen Sprunggelenks befindet und senkrecht zur anatomischen Fusslängsachse steht. Dadurch wird eine physiologische Plantarflexion und Dorsalextension ermöglicht und keine unnatürliche Bewegung aufgezwungen. Durch die biegesteife Gelenkverbindung können die bei einer Inversion oder Eversion im Fussgelenk entstehenden Zugkräfte und Druckkräfte über das Exoskelett auf die Laufsohle (via Schalenelemente) sowie auf den Unterschenkel (via Manschettenelement) übertragen werden und das Fussgelenk wird so geschützt. Das Drehgelenk ist vorzugsweise so gestaltet, dass es mit dem Stützelement in einer späteren Therapiephase ohne grossen Aufwand demontierbar ist. Um den maximal möglichen Öffnungswinkel zu begrenzen, kann das Drehgelenk mit bevorzugt zwei gegenüberliegenden, bogenförmigen Öffnungen versehen sein, welche als Führungsschiene für zwei gegenüberliegende, auf einem Gelenkdeckel montierte Stifte dienen, die mit Aufsetzen und Fixierung des Gelenkdeckels in die besagten Führungsschienen auf dem Drehgelenk versenkt werden. Je nach Länge der Führungsschiene und der Dimension der Stifte erfolgt nach bestimmter Drehung ein Anschlag durch die Stifte, welche so den maximalen Öffnungswinkel des Gelenks definieren. Bei gleichbleibender Führungsschiene ist so mit einem breiteren Stift ein kleinerer Öffnungswinkel möglich als mit einem schmaleren Stift. Um verschiedene Therapiephasen abdecken zu können, ist der Einsatz von verschiedenen Deckeln mit verschiedenen Stiften möglich, bevorzugt wird der gleiche Deckel dabei zweiseitig gestaltet, wobei jede Seite mit verschiedenen Stiftdimensionen versehen ist, so dass durch Umdrehen des Gelenkdeckels verschiedene Öffnungswinkel von vorzugsweise 0° - 50° ermöglicht werden. Für das Befestigen und Entfernen des Gelenkdeckels empfiehlt sich eine bereits im Gelenk integrierte Kopfschraube oder ein Klickaufsatz, welcher durch ein auf dem Gelenk befestigtes Zwischenstück angebracht werden kann. Das physiologische Gangbild soll durch die Endanschläge nicht beeinflusst werden, die Endanschläge im Drehgelenk bewirken daher nur bei extremeren Bewegungen ausserhalb der medizinischen einen Bewegungsstopp und dienen daher als sekundäre Einschränkung der Plantarflexion und Dorsalextension.Es sind vielfältige Gestaltungen des Exoskeletts denkbar. Um den Druck auf die empfindlichen Knöchel zu vermeiden wird vorzugsweise vorgeschlagen, das Exoskelett im Knöchelbereich auf der Innen- und Aussenseite so zu formen, dass mehr Platz verfügbar ist. Materialtechnisch werden für das Exoskelett am besten Kunststoff-Verbunde eingesetzt, bevorzugt Glas- oder Kohlefaserverbunde, welche die nötigen Steifigkeitseigenschaften bei geringem Eigengewicht erbringen. Als Kunststoff-Matrix kommen Epoxidharze und technische Thermoplaste wie Polyamid, Polyester, Polycarbonat etc. in Frage. Um das Leder oder Obermaterial des Schuhs gegen Reibung und Verschleiss zu schützen, kann das Stützelement auf der Innenseite mit einer Polsterung versehen werden, welche wiederum mit einem Klettverschluss am Schaft fixiert werden kann, dies um die Relativbewegung zwischen Exoskelett und Schuh zu verringern, bevorzugt unterstützt durch einen elastischen Spickel im Schaftmaterial direkt oberhalb der Fersenkappe. Die Polsterung verringert zudem den Druck des Exoskeletts auf den Schuhschaft bzw. das Bein und erhöht den Tragekomfort.

### Verschliessbänder

Bei der häufigsten Verletzung des Aussenbandapparates, dem Bänderriss des Ligamentum talofibulare anterius, ist neben der Einschränkung von Inversion und Eversion der Talusvorschub zu vermeiden, welcher bei einer Plantarflexion von über 20 Grad auftritt, und eine Zugbelastung auf das Ligamentum talofibulare anterius auslöst. Dies wird durch ein unelastisches, bevorzugt textiles Band gelöst, das am äusseren Schalenelement verankert in dieser Ausführung auf das Manschettenelement an der Fussinnenseite führt, wo es fixiert wird. Das unelastische vorzugsweise textile Band verbindet so das Schalenelement mit dem Stützelement und bremst oder limitiert die Drehbewegung nach hinten, d.h. es schränkt die Plantarflexion ein. Der Bandverlauf sorgt für einen zusätzlichen Schutz gegen den Talusvorschub, indem es genau über dem Talus verläuft und mittels Druck dessen Vorschubbewegung limitiert. Die Bandlänge zwischen den Ankerpunkten und damit die Begrenzung der Plantarflexion kann durch das verschieden starke Anziehen des Bandes angepasst werden. Zudem wird die Einschränkung der Inversion mit dieser Verbindung unterstützt, indem das Band die seitliche Auslenkung der Versteifungen verhindert.

Das Band hat vorzugsweise eine Breite im Bereich von 0.5 - 4 cm, insbesondere im Bereich von 1.5 - 3 oder 1.5 - 2.5 cm und umfasst ein geflochtenes Textilband aus Polyester, Polyamid, Baumwolle oder Fasermischungen. Das Band kann vorzugsweise an der Manschette über eine Klettverbindung, ggf. kombiniert mit einer Umlenkung, befestigt werden. Um eine vom Arzt vorgegebene und stets gleiche Einstellung zu gewährleisten ist es möglich, auf dem Band und/oder dem Schuh und/oder dem Exoskelett Markierungen anzubringen, die es dem Benutzer erlauben, stets die gleiche Länge des Bandes zwischen Schalenelement und Manschette einzustellen.

Wenn hier von einem dehnungsarmen oder unelastischen Band die Rede ist, ist generell darunter ein Band zu verstehen, das ein E-Modul von 1-5 GPa besitzt. Das Band ist dabei nicht als steife Struktur zu verstehen, sondern als biegeweiches Band, das nur in Zugrichtung in der Ebene des Bandes dehnungsarm ist.

Wenn im Zusammenhang mit dem Exoskelett von einem steifen Element, z.B. der Manschette oder dem Schalenelement gesprochen wird, so ist darunter eine Struktur zu verstehen, die im wesentlichen biegefest ist, d.h. in Richtung parallel zur Haupteben ein Biegemodul (DIN EN ISO 178) von wenigstens 30 GPa aufweist und in Richtung senkrecht dazu ein Biegemodul von wenigstens 20 GPa.

Bei sehr schweren Fussverletzungen ist neben dem Ligamentum talofibulare anterius auch das Ligamentum talofibulare posterior betroffen. Um dessen Zugbelastung zu vermeiden muss die Dorsalextension eingeschränkt werden. Dies ist durch ein möglichst dehnungsarmes, textiles Band gelöst, welches als Ankerpunkt in der Fersenkappe des Schuhs fest integriert ist und durch eine Öffnung im Manschettenelement umgeleitet und auf sich selbst fixiert wird. Die Fixierung kann an verschiedenen Ankerpunkten erfolgen, um den Abstand zwischen der Fersenkappe und dem Manschettenelement nach medizinischen Vorgaben zu regulieren. Bevorzugt erfolgt die Fixierung mittels Klettverschluss oder Druckknopf oder einer Kombination der beiden Verschlussmöglichkeiten um einer hohen Scherbelastung zu widerstehen. Ein Anziehen des Fusses und somit die Dorsalextension des Fusses ist durch diesen Mechanismus regulierbar.

### Spezialschuh

Neben den Stabilitätsanforderungen und Anpassungen an den Therapieverlauf muss der verletzte Fuss in einer möglichst neutralen Stellung, in der Regel bei 0°, in den Schuh einsteigen können. Ist dies nicht möglich, ist die verletzte Struktur am Fuss gefährdet. Dies wird beim hier vorgeschlagenen Schuh vorzugsweise durch eine weite Öffnung des Schuhs bis fast zur Schuhspitze gelöst. Der Schaft geht dabei im Vorfussbereich auf der Innen- und Aussenseite jeweils in eine Klapplasche über, welche den herkömmlichen Schuhschnitt mit spiegelgleichen Quartierteilen und Schuhlasche ersetzt. Die Klapplaschen lassen sich aufklappen und ermöglichen so dem Fuss einen neutralen Einstieg. Zum Verschliessen wird vorzugsweise zuerst die Klapplasche auf der Innenseite zugeklappt und danach die äussere, ein Klettverschluss sorgt für die gegenseitige Fixierung. In einer bevorzugten Ausführung der Erfindung ist in der äusseren Klapplasche zusätzlich ein elastisches Band integriert, welches zum Verschlusssystem gehört und einen Zug von der Fussaussenseite nach innen ausübt. Dies soll die Gefahr von Supination minimieren und sorgt für eine gewisse Stabilisierung des Vorfusses, besonders in der Abstossphase, in welcher der Fuss besonders gefährdet ist, ein Supinationstrauma zu erleiden. Das elastische Band tritt auf der Fussinnenseite aus der Klapplasche aus, wird wieder zur Aussenseite des Fusses umgelenkt und verläuft über den Rist zurück zur Fussaussenseite, wo es vorzugsweise mittels einer im äusseren Schalenelement integrierten Umlenkklammer oder -rolle nochmals umgelenkt wird und danach auf sich selbst mit Klettverschluss fixiert wird. Das Band besteht z.B. aus einer Fasermischung zusammengesetzt aus Polyester, Polyamid oder Baumwolle mit einem Elasthananteil von 20 - 40 %, um sich einerseits genügend an den Fuss anzupassen und andererseits trotzdem Stabilität zu gewährleisten.

Eine weitere mögliche Ausführungsform besteht darin, das elastische Band mit dem vorderen dehnungsarmen Textilband bevorzugt mittels einer Naht zu verbinden. Die Verbindung sollte dabei vor der Umlenkung stattfinden, damit durch die Umlenkung genügend Reibung entsteht, um als Ankerpunkt für das dehnungsarme Textilband zu dienen. Der Schliessmechanismus des Schuhs wird so vereinfacht, indem zwei separate Verschlusssysteme zu einem kombiniert werden.

Am oberen Schuhschaft im Beinbereich ist eine geteilte Zunge zu empfehlen um einen leichten Einstieg zu ermöglichen und die Schliessung zu erleichtern. Durch den Einsatz von weichem und bevorzugt gepolstertem Material werden Druckstellen vermieden.

Um die Öffnung zwischen der geteilten Zunge und den Klapplaschen zu schliessen befindet sich vorzugsweise eine kleine Lasche direkt oberhalb des Talus. Diese Lasche wird bevorzugt direkt in die Schnürung integriert, damit diese automatisch richtig platziert wird und ist nur einseitig befestigt, um einen ungehinderten Einstieg zu ermöglichen. Um den Talusvorschub auch noch mit der Schnürung zu unterbinden empfiehlt sich im Schaft jeweils ein Einschnitt auf der Innen- und Aussenseite, so kann ein verstärkter Druck auf den Talus ausgeübt werden. Die Lasche oberhalb des Talus sorgt zudem für eine Druckverteilung, um nicht punktuell Druckschmerzen zu verursachen.

Der Spezialschuh sollte bevorzugt aus weichen Materialien bestehen, damit sich der Schuh möglichst gut an den Fuss anpasst. Um den Komfort und die Atmungsfähigkeit zu erhöhen bietet sich der Einsatz von Ledermaterialien an.

### Verschlusssystem

Um eine einfache Bedienbarkeit zu erhalten sollte sich der Schuh mit wenigen Handgriffen verschliessen lassen. Im Fussbereich ist die Umlenkung für das elastische Band auf der Fussinnenseite daher bevorzugt mittels eines Klickverschlusses zu vereinfachen. Die Umlenkrolle mit dem männlichen Teil des Klickverschlusses ist dabei auf dem elastischen Band eingefädelt, der weibliche Teil des Klickverschlusses mit einem Riemen oder direkt am inneren Schalenelement befestigt. Durch diese Umsetzung ist das Verschliessen der Klapplasche mit einem Klick möglich und kann danach mittels Klettverschluss auf der Aussenseite oder bei einer Verbindung mit dem vorderen Textilband auf der Manschette auf der Fussinnenseite fein justiert und angezogen werden.

Das eigentliche Schnürsystem hat durch diese Umsetzung seinen Anfangspunkt erst oberhalb der Klapplasche bei den in den Schaft integrierten Einschnitten. Die Umsetzung des bekannten Schnürsystems mit einem Schuhsenkel und zwei freien Enden ist denkbar, wobei der Schuhsenkel bevorzugt nur auf einer Seite durch eine oder zwei Ösen eingefädelt wird und danach Haken zum Einsatz kommen, um das Einfädeln zu erleichtern und vor allem den Einstieg nicht durch einen bereits eingefädelten Schnürsenkel zu behindern. Eine besondere Ausführungsform ist jedoch eine Schnürung, welche das oben erwähnte Schnürsystem auch in ein einhändiges Schnürsystem umwandeln lässt und so der Patient zwischen zwei Varianten wählen kann. Dabei wird der bestehende Schuhsenkel auf die richtige Länge gekürzt und dann auf einer Seite fixiert, am besten durch eine Verknüpfung in eine Öse. Die Schnürung besteht so nicht aus zwei Schnurenden, sondern nur aus einem Schnurende und der Schuh ist mit einer Hand schliessbar, indem der Schnürsenkel mittels der Haken nach oben geführt und am Schaftabschluss fixiert wird. Die Fixierung kann unter anderem durch einen Klett oder Abschlusshaken erfolgen und/oder mittels Reibung durch mehrmaliges Umrunden einer oder mehrere Haken oder einem Klemmverschluss.

Die oben erwähnte Zwischenlasche oberhalb des Talus ist vorzugsweise in den

Schuhbändel integriert, indem der Schuhsenkel durch eine Öffnung in der Lasche oder aufgenähten Kanal durchgefädelt wird.

### Bandage

Eine mögliche Ausführung der Erfindung ist die Ausführung als Bandage mit einem enganliegenden Textil ohne Zehenteil und mit geschlossener oder freiliegender Ferse in Kombination mit einem Exoskelett mit den für die Schuhanwendung erwähnten (Material)Eigenschaften. Die Bandage sollte aus weichen und leicht elastischen Materialien bestehen, damit eine möglichst gute Anpassung an den Fuss gewährleistet ist. Das drucksteife Exoskelett umfasst wie in der Schuhausführung ein Stützelement und ein Schalenelement verbunden über ein inneres und äusseres Drehgelenk. In der Bandagenausführung ist jedoch das Schalenelement durch eine Fussschale erweitert, welche bevorzugt von der Ferse bis vor die Zehengrundgelenke führt, jedoch von der Ferse her gesehen mindestens die Hälfte der Fusslänge abdeckt. Auf der äusseren Seite ist das Schalenelement im Vergleich zur inneren Seite des Schalenelements bevorzugt weiter nach vorne Richtung kleine Zehe gezogen, um den Fuss beim Abrollvorgang länger zu führen und zusätzlich lateral zu schützen. Es ist jedoch auch eine symmetrische Ausführung denkbar, welche sowohl auf den linken wie den rechten Fuss anwendbar ist. Das druckstabile und zugstabile Stützelement sowie die Drehgelenke mit Endanschlägen sind wie bei der oben beschriebenen Schuhanwendung ausgeführt. Das Drehgelenk ermöglicht so auch in dieser Ausführung eine physiologische Bewegung im Sprunggelenk, begrenzt durch einen definierten Bewegungsspielraum im Gelenk. Durch die biegesteife Gelenkverbindung können die bei einer Inversion oder Eversion im Fussgelenk entstehenden Zugkräfte und Druckkräfte über das Stütz- und Schalenelement auf die Fussschale sowie auf den Unterschenkel (via Manschettenelement) übertragen werden und das Fussgelenk wird so geschützt.

Die primäre Begrenzung der Dorsalextension wird wie oben dargestellt durch ein nachgelagertes, möglichst dehnungsarmes, textiles Band gelöst, welches als Ankerpunkt in der Fussschale verklebt ist und durch eine Öffnung im Manschettenelement umgeleitet und auf sich selbst fixiert wird. Die Fixierung kann an verschiedenen Ankerpunkten erfolgen, um den Abstand zwischen der Fussschale und Manschette nach medizinischen Vorgaben zu regulieren. Bevorzugt erfolgt die Fixierung mittels Klettverschluss oder Druckknopf oder einer Kombination der beiden, um auch einer hohen Scherbelastung zu widerstehen.

Ein neutraler Einstieg ist auch bei der Bandage Voraussetzung und daher ist die komplette Öffnung der Bandage notwendig. Dies kann wie bei der Schuhanwendung über zuklappbare Seitenteile gelöst sein, vorzugsweise über zwei von der Innenseite respektive der Fussaussenseite zur Ristmitte und wenigstens bereichsweise überlappende Seitenteile. Auf der Aussenseite kann zusätzlich ein vorzugsweise dehnungsarmes, textiles Textilband eingenäht sein, welches zum Verschlusssystem gehört und einen Zug von der Fussaussenseite nach innen ausübt. Dies kann die Gefahr von Supination minimieren und sorgt für eine gewisse Stabilisierung des Vorfusses, besonders in der Abstossphase, in welcher der Fuss besonders gefährdet ist, ein Supinationstrauma zu erleiden. Das Band tritt auf der Fussinnenseite aus und wird durch eine auf dem Schalenelement befestigte Umlenkrolle wieder zur Aussenseite des Fusses umgelenkt. Von der Aussenseite führt das Textilband erneut über eine am Schalenelement fixierte Umlenkrolle nach oben auf die Innenseite der Manschette und wird dort vorzugsweise mittels Klettverschluss oder ggf. kombiniert mit wiederum einer Umlenkung fixiert. Das Band verbindet so das Schalenelement mit dem Stützelement und limitiert beim Gehen die Drehbewegung nach hinten, d.h. es schränkt die Plantarflexion ein. Zusätzlich sorgt der Bandverlauf für einen Schutz gegen den Talusvorschub, indem es genau über dem Talus verläuft und mittels Druck dessen Vorschubbewegung limitiert. Die Bandlänge zwischen den Ankerpunkten und damit die Begrenzung der Plantarflexion kann durch das verschieden starke Anziehen des Bandes angepasst werden. Zudem wird die Einschränkung der Inversion mit dieser Verbindung unterstützt, indem das Band die seitliche Auslenkung der Versteifungen verhindert.

Das dehnungsarme, textile Band hat die analogen oder gleichen Materialeigenschaften wie bei der Schuhausführung und ebenfalls vorzugsweise eine Breite im Bereich von 0.5 - 4 cm, insbesondere im Bereich von 1.5 - 3 oder 1.5 - 2.5 cm. Um eine vom Arzt vorgegebene und stets gleiche Einstellung zu gewährleisten, ist es möglich, auf dem Band und/oder der Bandage und/oder dem Exoskelett Markierungen anzubringen, die es dem Benutzer erlauben, stets die gleiche Länge des Bandes zwischen Schalenelement und Manschette einzustellen.

Im Beinbereich ist für die komplette Öffnung eine geteilte Zunge zu empfehlen. Um die Öffnung zwischen der geteilten Zunge und dem Fussbereich zu schliessen, befindet sich vorzugsweise eine kleine Zwischenlasche direkt oberhalb des Talus. Diese Lasche wird bevorzugt direkt in die Schnürung integriert, damit diese automatisch richtig platziert wird, und ist nur einseitig befestigt, um einen ungehinderten Einstieg zu ermöglichen. Die Zwischenlasche oberhalb des Talus sorgt für einen Widerstand gegen den Talusvorschub und zudem für eine Druckverteilung, um nicht punktuell Druckschmerzen zu verursachen. Die Schnürung der Bandage kann dabei wie in der Schuhausführung angewendet werden. Nach Bedarf kann das gesamte Exoskelett in einer späteren Therapiephase von der Bandage entfernt werden, indem das vordere textile dehnungsarme Band nicht mehr via das Schalenelement umgelenkt wird und so die Verbindung mit der Bandage nicht mehr gewährleistet ist. Nach Entfernung des Exoskeletts wird das textile Band nicht mehr umgelenkt, sondern wie gehabt mit dem äusseren Seitenteil nach innen geklappt und dann vorzugsweise unter dem Fuss durchgeführt und von der Innenseite her nach oben gezogen und auf der Beininnenseite durch einen Klettverschluss fixiert. So kann die Bandage in einer zweiten Phase als leichte Stütze verwendet werden.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine laterale Ansicht eines orthopädischen Stabilschuhs mit geschlossenen Bändern;
- Fig. 2: eine laterale Ansicht gemäss Fig. 1 mit auf der lateralen Seite geöffneten Bändern;
- Fig. 3: in a) eine mediale Ansicht mit Blickwinkel leicht von oben auf ein Ausführungsbeispiel gemäss Fig. 1 mit teilweise geöffneten Bändern und ohne Schuhbändel (Schnürung); in b) eine mediale Ansicht mit Blickwinkel leicht von oben auf ein weiteres Ausführungsbeispiel;
- Fig. 4: eine dorsale Ansicht gemäss Fig. 1 inklusive detailliertem Gelenkaufbau ohne Gelenkanschlag;
- Fig. 5: eine perspektivische Detailzeichnung des lateralen Gelenks mit Gelenkanschlag mit Blick von vorne;
- Fig. 6: eine perspektivische Detailzeichnung des lateralen Gelenks mit Gelenkanschlag ohne Gelenkdeckel und Gegenplatte mit Blick von vorne;
- Fig. 7: eine laterale Ansicht einer Bandage mit Exoskelett;

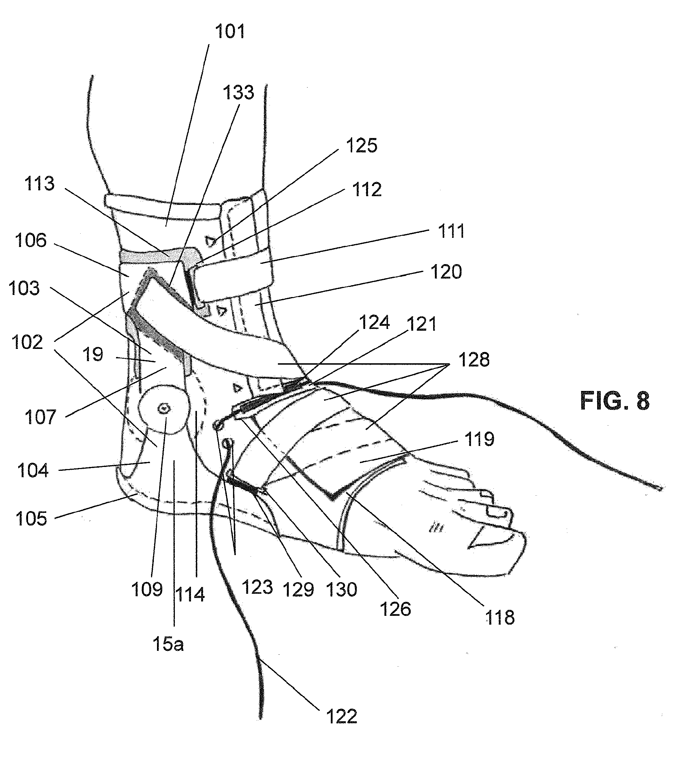

- Fig. 8: eine mediale Ansicht der Bandage mit Exoskelett leicht von oben mit teilweise geöffneter Schnürung;
- Fig. 9: eine perspektivische Explosionsdarstellung des lateralen Gelenks gemäss einem weiteren Ausführungsbeispiel mit Gelenkanschlag mit Blick vom Fuss; und
- Fig. 10: eine Explosions-zeichnung des lateralen Gelenks gemäss dem weiteren Ausführungsbeispiel mit Gelenkanschlag mit Blick von vorne

### BESCHREIBUNG BEVORZUGTER AUSFÜHRUNGSFORMEN

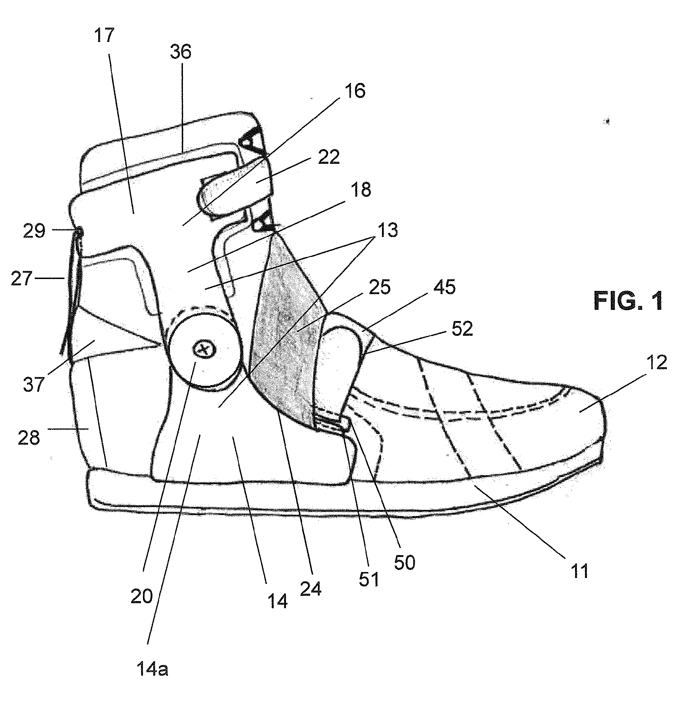

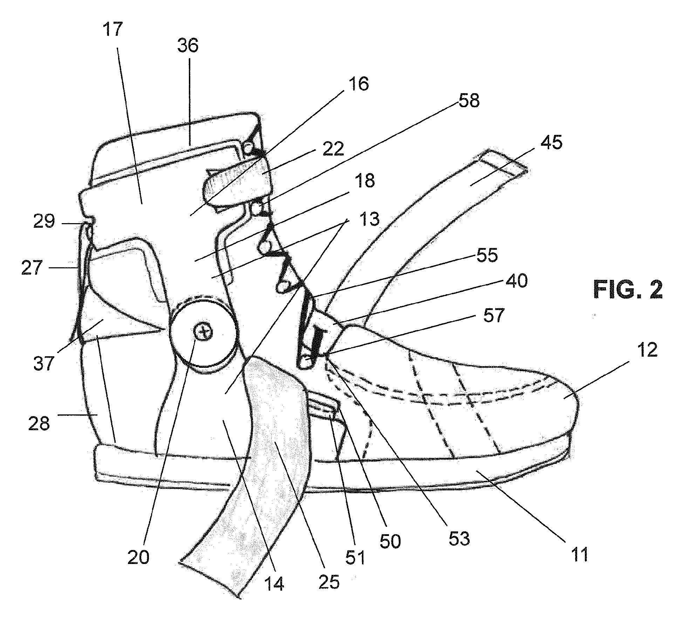

Der in Fig. 1 bis 5 dargestellte orthopädische Stabilschuh mit Laufsohle 11 und Schaft 12, besitzt ein verstärkendes Exoskelett, gesamthaft bezeichnet als 13. Das Exoskelett 13 bildet das stabile Gerüst und besteht aus drei Elementen: einem äusseren Schalenelement 14 und einem inneren Schalenelement 15, sowie einem Stützelement 16, wobei das Stützelement 16 in eine den Unterschenkel umfassende Manschette 17 mit längsförmigen Versteifungen auf der Fussaussenseite 18 und der Fussinnenseite 19 aufgeteilt werden kann. Die Schalenelemente 14 und 15 sind jeweils durch ein äusseres Drehgelenk 20 und ein inneres Drehgelenk 21 mit den längsförmigen Versteifungen 18 und 19 verbunden. Mittels dieser stabilen Gelenkverbindung können die bei einer Inversion oder Eversion im Fussgelenk entstehenden Zugkräfte und Druckkräfte über das Exoskelett 13 auf die Laufsohle (via Schalenelemente 14, 15) sowie auf den Unterschenkel (via Manschette 17) übertragen werden, das Fussgelenk wird so seitlich stabilisiert und vor Verletzungen geschützt.

In der dargestellten Ausführungsform sind die beiden Schalenelemente 14 und 15 zwischen Schaft 12 und Laufsohle 11 verklebt und unmittelbar unter der nicht dargestellten Brandsohle angeordnet um möglichst nahe am Fuss zu sein. Das äussere Schalenelement 14 ist in dieser Ausführung zudem etwas länger angefertigt um den Fuss seitlich beim Abrollvorgang zu führen.

Die Manschette 17 umfasst den Schaft 12 oberhalb der beiden Knöchel und kann durch ein Verschlusselement 22 in Form eines Verschlussbandes rund um den Unterschenkel verschlossen werden. Dabei handelt es sich in dieser Ausführung um einen Klettverschluss mit Umlenkrolle 23 zur einhändigen Bedienung.

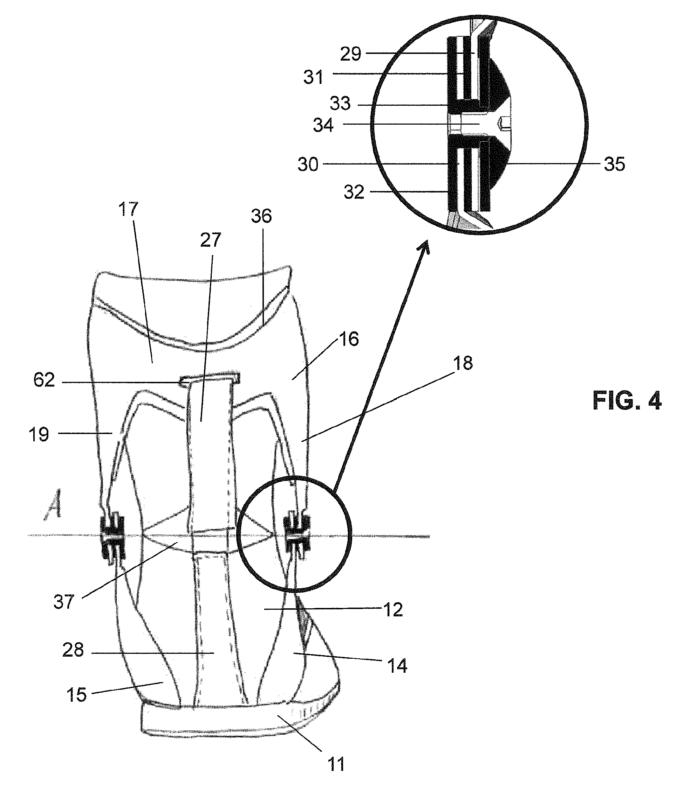

Wie in Fig. 4 ersichtlich, überlappen sich die längsförmigen Versteifungen 18 und 19 mit den beiden Schalenelementen 14 und 15 und bilden so jeweils die äussere 29 und innere Gelenkfläche 30 der Drehgelenke 20 und 21. Um die Reibung bei Drehbewegungen zu minimieren ist zwischen den Gelenkflächen eine PTFE-Scheibe 31 integriert. Auf der Innenseite, fussseitig, befindet sich eine Gegenplatte 32, welcher mit einem Innengewindeelement 33 für die Gelenksachse versehen ist. Die Gelenksachse wird bei dieser Ausführung mit einer Gelenkschraube 34 gebildet, die im Gelenksdeckel 35 auf der Aussenseite versenkt ist. Das Stützelement 16 ist somit demontierbar und kann gegen Ende der Therapie entfernt werden um die seitliche Stabilisierung zu minimieren und nur noch durch die Schalenelemente 14 und 15 aufrechtzuerhalten. Die Drehgelenksachse A befindet sich dabei möglichst nahe bei der natürlichen Gelenksachse, leicht unterhalb des äusseren Knöchels.

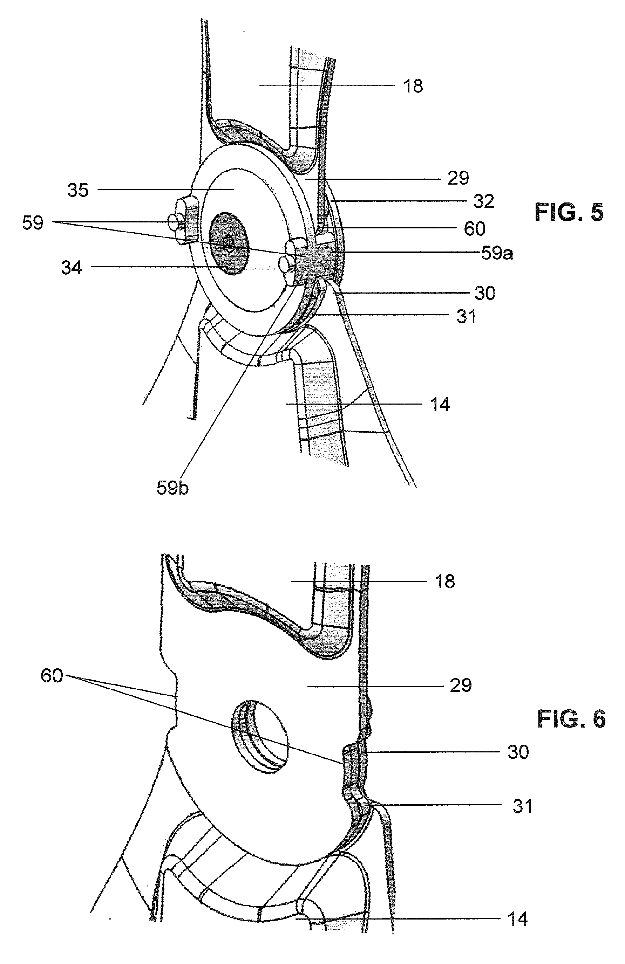

Fig. 5 und 6 zeigen detaillierte perspektivische Ansichten der Gelenkmechanik. Die äussere Gelenkfläche 29 und innere Gelenkfläche 30 sowie die PTFE-Scheibe 31 im Gelenk sind jeweils mit zwei gegenüberliegenden bogenförmigen Ausnehmungen 60 versehen. Der Gelenkdeckel 35 lässt sich beidseitig via Gelenkschraube 34 auf dem Gelenk fixieren und ist mit zwei sich gegenüberliegenden Stiften 59 versehen, wobei sich die Stifte 59 auf der einer Deckelseite verglichen mit der anderen Seite im Durchmesser wenigstens in einem Bereich verjüngen. Konkret ist in der vorliegenden Situation ein paar von Stiften 59a bezüglich Drehachse auf beiden Seiten des Gelenksdeckels 35 angeordnet, diese Stifte zeigen zum Fuss und damit greifen sie in die Ausnehmungen 60 ein. Diese Stifte haben eine in Drehrichtung des Gelenks grosse Länge und führen dadurch zu einer geringen Beweglichkeit um das Gelenk. Auf der anderen Seite bezüglich Deckelebene ist an der gleichen Position bezüglich Drehachse ein weiteres Paar von Stiften 59b angeordnet. Diese Stifte verfügen nun aber in jenem Bereich, in welchem sie, wenn die Deckelplatte 35 in umgekehrter Position aufgeschraubt ist und damit die Stifte 59b zum Fuss zeigen, in die Ausnehmungen in der Gelenkfläche 30 eingreifen, über eine geringere Länge in Drehrichtung des Gelenks. Dies wird realisiert, indem der Stift 59b gestuft ausgebildet ist und an der Spitze einen geringeren Durchmesser in Drehrichtung des Gelenks aufweist. In dieser Situation mit umgekehrt aufgesetztem Gelenksdeckel 35 ist der Beweglichkeitsbereich des Gelenks grösser. Wenn die Seite mit dem breiteren Teil 59a der Stifte 59 auf das Gelenk und in die Ausnehmungen 60 gesetzt wird, ist daher ein kleinerer Öffnungswinkel möglich, als wenn der Gelenkdeckel 35 umgekehrt als in Figur 5 dargestellt mit den im Durchmesser kleineren Stiften 59b auf das Gelenk und die Ausnehmungen 60 montiert wird. Durch die verschieden grosse Stiftbreite in Umfangsrichtung auf der Innen- bzw. Aussenseite des Gelenkdeckels 35 sind so verschiedene Endanschläge für den Bewegungsspielraum je nach Therapiephase realisierbar. Wird der Gelenkdeckel 35 weggelassen, lässt sich das Gelenk frei bewegen, soweit die Beweglichkeit nicht durch die Bänder begrenzt ist.

Um den Komfort zu erhöhen und Druck auf empfindliche Stellen zu vermeiden ist die Form des Exoskeletts 13 entscheidend. Im Bereich der Knöchel besitzt das Exoskelett eine leicht konkave Ausbuchtung. Dies gibt den Knöcheln mehr Platz, vor allem für die bei Verletzungen häufig vorkommenden Schwellungen. Gegen oben weist die Manschette 17 einen konvexen Abschluss auf, um einen punktuellen Druck auf den Unterschenkel zu vermindern. Zusätzlich ist das Stützelement grösstenteils mit einer Polsterung 36 versehen, ebenfalls zur Druckverminderung. Um das Schaftmaterial des Schuhs gegen Reibung und Verschleiss zu schützen und die Drehgelenkbewegung möglichst direkt über den Schaft auf das Fussgelenk/Bein zu übertragen, ist die Polsterung 36 mittels Klettverschluss am Schuh fixiert, um eine Relativbewegung zwischen Schuh und Stützelement zu minimieren. Ein elastischer, in den Schaft eingenähter Spickel 37 oberhalb der Fersenkappe 28 sorgt ebenfalls für eine möglichst geringe Relativbewegung.

Materialtechnisch wurde bei dieser Ausführung ein steifer Kohle-Glasfaser-Verbund gewählt, mit zusätzlich verstärkten Gelenkstrukturen. Dies erbringt die nötige Steifigkeit bei einem sehr geringen Eigengewicht.

Zwischen dem äussere Schalenelement 14 und dem Schaft 12 verklebt, befindet sich ein dem Drehgelenk vorgelagertes, dehnungsarmes, textiles Band 25. Das Band führt auf die mediale Seite des Stützelements 16 und wird am Anbindungsfeld 26 durch einen Klett fixiert. Dadurch entsteht eine Verbindung zwischen dem Schalenelement 14 mit dem Stützelement 16, welche die Drehbewegung der Drehgelenke 20 und 21 nach vorne limitiert und somit die Plantarflexion einschränkt. Die Stärke der Limitierung hängt vom Abstand der Ankerpunkte 24 und 26 ab, welcher sich durch verschieden starkes Anziehen des Bandes 25 regulieren lässt. Dank dem Verlauf über den Talus wird auch dessen Vorschubbewegung begrenzt und eine unerwünschte Dehnung des Ligamentum talofibulare anterius verhindert. Zusätzlich verhindert das Band eine seitliche Auslenkung des Exoskeletts 13 nach aussen und unterstützt damit die Einschränkung der Inversion/Supinationsbewegung. Wird das Stützelement 16 entfernt, kann das Band 25 durch einen Klettverschluss direkt an den Schaft fixiert werden.

Nachgelagert befindet sich ein weiteres, unelastisches Band, beschriftet mit der Zahl 27. Das Band schränkt ein Anziehen des Fusses und damit die Dorsalextension ein, indem es an der Fersenkappe 28 fixiert ist und zur Manschette 17 führt. Auf der Manschette 17 wird das Band 27 in einem eingearbeiteten Schlitz 62 umgelenkt und danach auf sich selbst fixiert. Je nachdem an welcher Stelle das Band 27 fixiert wird, wird der Abstand zwischen Fersenkappe und Manschette verändert und damit die Dorsalextension begrenzt oder zugelassen. Nach Entfernung des Stützelements 16 kann das Band 27 als Anziehhilfe verwendet oder abgeschnitten werden.

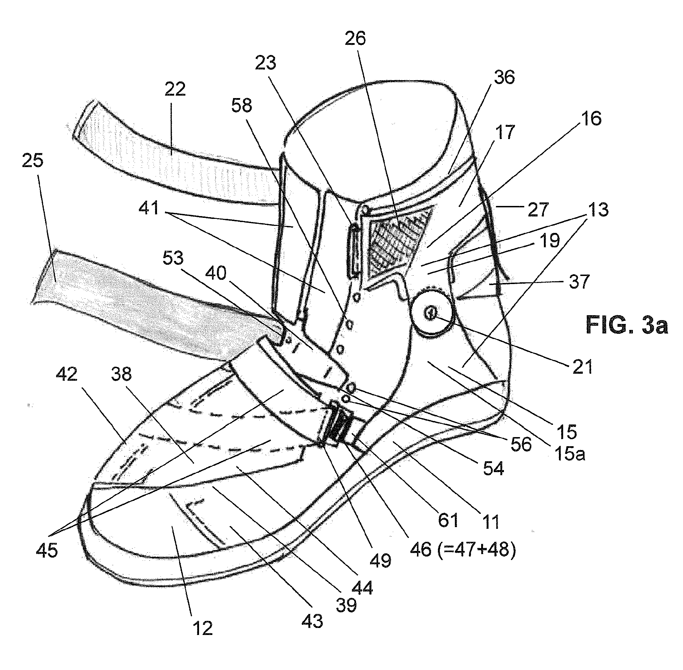

In Fig. 3a am besten ersichtlich ist das Einstiegsystems dieser Ausführung mit den Klapplaschen 38 und 39, der Zwischenlasche 40 und der geteilten Zunge 41. Das System ermöglicht eine komplette Öffnung sowie den Einstieg mit neutraler Fussstellung und ist damit ein wichtiger Bestandteil dieser Erfindung um die medizinischen Bewegungsvorgaben auch beim Anziehen des Schuhs einzuhalten.

Die Klapplaschen 38 und 39 sind eigentliche Verlängerungen aus den Quartierteilen 42 und 43 des Schafts 12. Zum Verschliessen wird zuerst die innere Klapplasche 39 eingeklappt und danach die äussere Klapplasche 38 auf der inneren mittels Klettverschluss 44 fixiert. In die äussere Klapplasche 38 integriert ist ein elastisches Band 45. Dieses verläuft vom Fussaussenrand nach innen, wo es aus der äusseren Klapplasche 38 austritt. Der Verlauf des Bandes 45 von vorne lateral nach hinten medial stabilisiert den Vorfuss in der Abstossphase. Auf der Innenseite wird das Band 45 umgelenkt und führt dann wieder zur Aussenseite. Um die Lasche einfach Verschliessen zu können, ist auf der inneren Seite ein Klickverschluss 46 angebracht, bestehend aus einem weiblichen Teil 47 und einem männlichen Teil 48. Der männliche Teil 48 des Klickverschlusses 46 ist dabei mittels einer Umlenkrolle 49 am elastischen Band 45 befestigt, damit das Band frei durchlaufen kann. Der weibliche Teil 47 ist durch einen Riemen 61auf dem inneren Schalenelement 15 fixiert. Auf der Aussenseite wird das Band 45 nochmals umgelenkt via Umlenkrolle 50. Die Befestigung der Umlenkrolle 50 ist durch einen Riemen 51 gelöst, welche zwischen Schaft 12 und dem äusseren Schalenelement 14 verklebt ist. Das Band 45 wird nach der Umlenkung auf sich selbst mit einem Klettverschluss 52 angezogen und befestigt. Durch den Klickverschluss 46 kann der Schuh mit einem Griff schnell verschlossen werden und danach durch den Klettverschluss 52 nachjustiert werden. Im Beinbereich des Schaftes 12 ist eine geteilte Zunge 41 umgesetzt. Dies ermöglicht eine komplette Öffnung und besteht aus weichem Material um Druckstellen und Falten zu vermeiden. Um den Abstand zwischen der geteilten Zunge 41 und den Klapplaschen 38 und 39 zu überbrücken ist eine Zwischenlasche 40 einseitig auf der Innenseite angenäht. Damit eine Platzierung oberhalb des Talus gewährleistet ist, ist die Zwischenlasche 40 in die Schnürung integriert. Dadurch wird der Talusvorschub via Schnürung zusätzlich verringert. Die Einschnitte auf der Aussenseite 53 und Innenseite 54 im Schaft verstärken diesen Effekt.

Die Schnürsenkel 55 werden auf der Innenseite in die Ösen 56 eingefädelt sowie via Kanal durch die Zwischenlasche 40 geführt. Die doppelten Ösen 56 sorgen dabei für eine breitere Zugverteilung. Auf der Aussenseite befindet sich ein Bremshaken 57, wo der Schnürsenkel 55 fixiert wird. Danach werden beide Enden des Schnürsenkels 55 via sechs bis acht Haken 58 nach oben geführt und durch einen Schnürknoten fixiert.

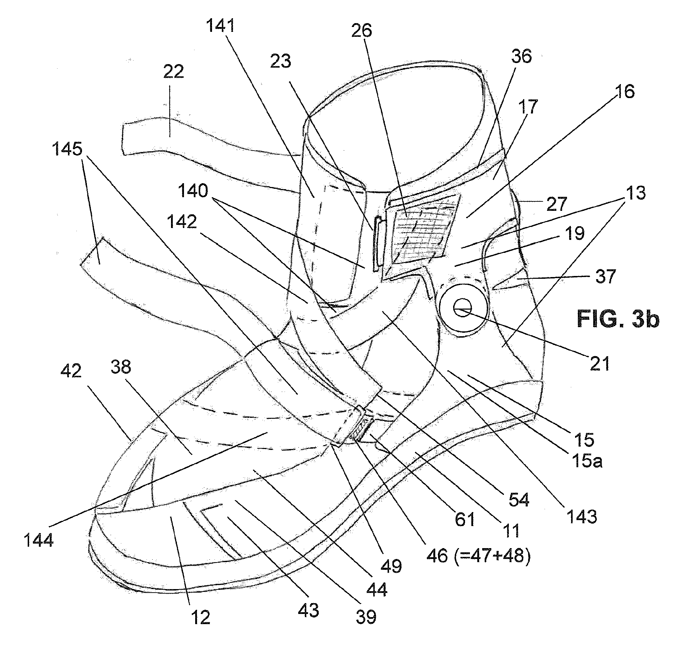

Fig. 3b zeigt in einer Darstellung gemäss Fig. 3a ein weiteres Ausführungsbeispiel mit etwas anderer Ausgestaltung der Führung der Bänder und des Einstiegsbereichs. Soweit in den Fig. 3a und 3b gleiche Bezugszeichen verwendet werden so bezeichnen diese die analogen Merkmale im der jeweiligen Ausführungsbeispiel.

In der Ausführung gemäss Fig. 3b ist die Schnürung durch ein Klettsystem ersetzt. Konkret sind in der vorliegenden Situation im Beinbereich des Schaftes 12 statt einer geteilten Zunge ein bis zwei breite Textil-Kletten 140, 141 aus weichem, dehnungsarmem Material integriert, um Druckstellen zu vermeiden. Die Textilkletten setzen sich aus einer inneren Textilklette 140 und einer äusseren Textilklette 141 zusammen. Die inneren Textilkletten 140 sind auf der Oberseite mit Klettverschluss-Flausch ausgestattet, die äusseren Textilkletten 141 auf der Unterseite mit Klettverschluss-Haken. Beim Verschliessen des Schuhs werden die äusseren Kletten 141 auf den inneren Kletten mittels Klettverschluss fixiert.

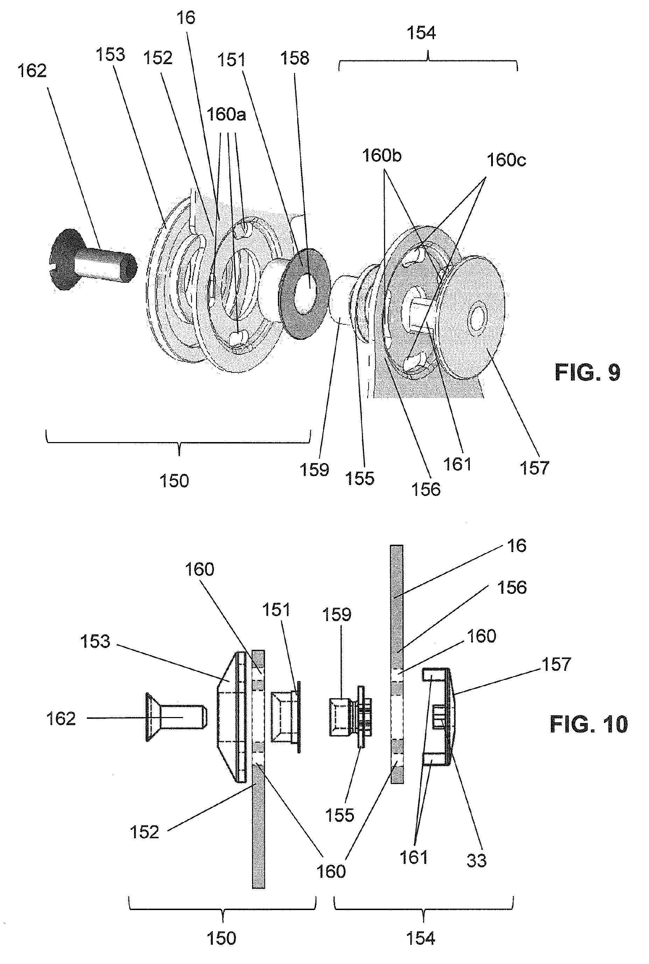

Aus dem Einschnitt innen 54 und Einschnitt aussen 53 verlaufen zwei dehnungsarme, textile Kreuz-Bänder 142 und 143. Das äussere dehnungsarme, textile Kreuzband 142 führt vom Einschnitt aussen 53 schräg nach oben und wird an der medialen Seite auf dem Anbindungsfeld 26 mittels Klettverschluss fixiert. Das innere dehnungsarme, textile Kreuzband 143 verläuft vom Einschnitt innen 54 schräg nach oben und wird an der lateralen Seite auf der Manschette 17 ebenfalls auf einem Anbindungsfeld (nicht sichtbar in Fig. 3b) mittels Klettverschluss fixiert. Durch die Kreuzung der beiden dehnungsarmen textilen Kreuzbänder 142 und 143 entsteht eine zusätzliche Stabilität und der Talusvorschub wird verstärkt unterbunden. Wird die Stütze 16 entfernt, erfolgt die Fixierung der beiden textilen dehnungsarmen Kreuzbänder 142 und 143 direkt unter der ehemaligen Manschette 17 im Beinbereich des Schafts 12 ebenfalls mit Klettverschluss. Ist die Ausführung mit der Gelenkvariante aus Fig. 9 ausgestattet, besteht die Möglichkeit, in einem ersten Schritt durch Abklappen der Stütze 16 die beiden dehnungsarmen, textilen Kreuzbänder 142 und 143 direkt unter der Manschette 17 zu fixieren und danach die Stütze wieder nach oben zu klappen. Dies hat den Vorteil, dass die Manschette 17 durch den Druck die Fixierung verstärkt.

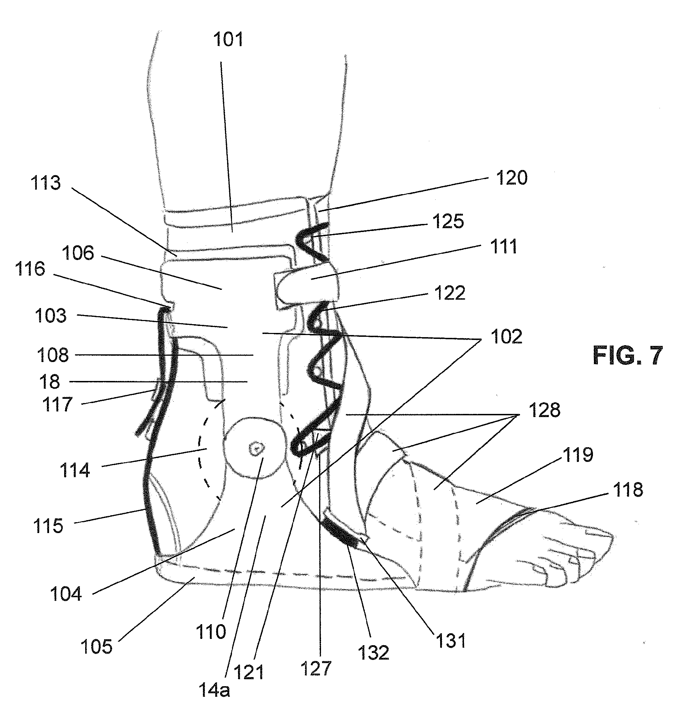

Um das Schliessen des Schuhs zu vereinfachen, sind das in die äusserer Klapplasche 38 integrierte elastische Band 144 sowie das dehnungsarme, vordere Textilband 145 bei dieser Ausführung durchgängig geführt. Dabei ist das vordere dehnungsarme Textilband 145 gleich nach Austritt aus der äusseren Klapplasche 38 an das elastische Band 144 angenäht und wird auf der Aussenseite mittels Umlenkrolle (nicht sichtbar in Fig. 3b) nach oben gelenkt und im Anbindungsfeld 26 medial fixiert. Das Anbindungsfeld 26 ist dabei so dimensioniert, dass sowohl das dehnungsarme Textilband 145, wie auch das äussere dehnungsarme, textile Kreuzband 143 fixiert werden können. Fig. 6 und 7 zeigen eine Bandage 101 aus Textil verbunden mit einem Exoskelett 102 hergestellt aus einem Kohle-Glasfaser-Verbund. Das Exoskelett 102 setzt sich aus einem Stützelement 103 und einem Schalenelement 104 zusammen, wobei das Schalenelement 104 mit einer Fussschale 105 verbunden ist. Das Stützelement 103 wiederum besteht aus einer Manschette 106 und jeweils einer innenseitig und aussenseitig längsförmigen Versteifung 107 und 108. Stützelement 103 und Schalenelement 104 sind jeweils über ein inneres Drehgelenk 109 und äusseres Drehgelenk 110 verbunden, welche den Drehgelenken in Fig. 4 bis 6 entsprechen. Die Manschette 106 umfasst das Bein oberhalb der beiden Knöchel und kann durch ein Verschlusselement 111 in Form eines Verschlussbandes rund um den Unterschenkel verschlossen werden. Dabei handelt es sich in dieser Ausführung um einen Klettverschluss mit Umlenkrolle 112 zur einhändigen Bedienung.

Um den Komfort zu erhöhen und Druck auf empfindliche Stellen zu vermeiden, ist die Form des Exoskeletts 102 entscheidend. Im Bereich der Knöchel besitzt das Exoskelett 102 eine leicht konkave Ausbuchtung. Dies gibt den Knöcheln mehr Platz, vor allem für die bei Verletzungen häufig vorkommenden Schwellungen. Gegen oben weist die Manschette 106 einen konvexen Abschluss auf, um einen punktuellen Druck auf den Unterschenkel zu vermindern. Zusätzlich ist das Stützelement 103 grösstenteils mit einer Polsterung 113 versehen, ebenfalls zur Druckverminderung. Um das Textil der Bandage gegen Reibung und Verschleiss zu schützen und die Drehgelenkbewegung möglichst direkt auf das Fussgelenk/Bein zu übertragen, ist die Polsterung 113 mittels Klettverschluss auf der Bandage 101 fixiert um eine Relativbewegung zwischen Bandage 101 und Stützelement 103 zu minimieren. Weiter besitzt die Bandage 101im Bereich der Knöchel eine eingenähte Polsterung aus Schaumstoff 114, um die verletzten Strukturen zu schützen. Nachgelagert befindet sich ein unelastisches Band 115, welches ein Anziehen des Fusses und damit die Dorsalextension einschränkt, indem es an der Fussschale 105 fixiert ist und zur Manschette 106 führt. Auf der Manschette 106 wird das Band 115 in einem eingearbeiteten Schlitz 116 umgelenkt und danach auf sich selbst durch einen Druckknopf 117 fixiert. Je nachdem an welcher Andockstelle das Band 115 fixiert wird, wird der Abstand zwischen Fussschale und Manschette verändert und damit die Dorsalextension reguliert.

Die komplette Öffnung, damit der Fuss neutral in die Bandage 101 einsteigen kann, wird im Ristbereich durch klappbare Seitenteile 118 (innen) und 119 (aussen) realisiert, sowie einer geteilten, aus weichen Textilien bestehende Zunge 120 im Beinbereich. Eine innenseitig befestigte Zwischenlasche 121 schliesst dabei die Öffnung zwischen dem Rist- und Beinbereich.

Zur Schliessung der Bandage 101 befindet sich anschliessend an die klappbaren Seitenteile 118 und 119 eine Schnürung. Startend in den zwei Ösen 123 wird ein Schnürsenkel 122 durch einen Kanal 124 in der Zwischenlasche 121 geführt, um diese richtig zu positionieren und durch die Schnürung einen Druck auf den Talus auszuüben. Dieser Druck wird zudem durch die integrierten Einschnitte innen 126 und aussen 127 verstärkt. Danach wird der Schnürsenkel via Haken 125 nach oben geführt und verknotet.

Im Ristbereich wird zuerst das innere Seitenteil 118 eingeklappt und danach das äussere Seitenteil 119 auf dem inneren Seitenteil 118 mittels Klettverschluss fixiert. In das äussere Seitenteil 119 integriert ist ein dehnungsarmes, textiles Band 128. Dieses Band 128 verläuft vom Fussaussenrand nach innen, wo es aus dem äusseren Seitenteil 119 austritt. Der Verlauf des Bandes 128 von vorne lateral nach hinten medial stabilisiert den Vorfüss in der Abstossphase. Auf der Innenseite wird das Band 128 durch eine Umlenkrolle 129 umgelenkt und führt dann wieder zur Aussenseite. Die Umlenkrolle 129 ist dabei via Riemen 130 direkt mit dem Schalenelement 104 verbunden. Auf der Aussenseite wird das Band 128 erneut via Umlenkrolle 131 umgelenkt, wobei die Umlenkrolle 131 ebenfalls durch einen Riemen 132 auf dem Schalenelement 104 fixiert ist. Das Band 128 verläuft danach weiter nach oben auf die Innenseite des Stützelements 103 und wird auf der Manschette 106 mittels Klettverschluss am Anbindungsfeld 133 fixiert. Durch den Verlauf des Bandes 128 entsteht eine Verbindung zwischen dem Schalenelement 104 mit dem Stützelement 103, welche die Drehbewegung der Drehgelenke 109 und 110 nach vorne limitiert und somit die Plantarflexion einschränkt. Die Stärke der Limitierung hängt vom Abstand der Umlenkrolle 131 und dem Anbindungsfeld 133 ab, welcher sich durch verschieden starkes Anziehen des Bandes 128 regulieren lässt. Dank dem Verlauf über den Talus wird auch dessen Vorschubbewegung begrenzt. Zusätzlich verhindert das Band eine seitliche Auslenkung des Exoskeletts 102 nach aussen und unterstützt damit die Einschränkung der Inversion/Supinationsbewegung.

Das Exoskelett 102 ist einerseits durch das Band 128 mit der Bandage 101, andererseits mit dem Klettverschluss auf der Polsterung 113 des Stützelements 103 verbunden. In einer späteren Therapiephase ist das komplette Exoskelett 102 somit einfach entfernbar. Zur Schliessung der Bandage 101 ohne Exoskelett 102 bleibt die Schnürung bestehen, das textile Band 128 wird jedoch nicht mehr durch Umlenkrollen 129 und 131 geführt, sondern wie gehabt mit dem äusseren Seitenteil 119 nach innen geklappt, danach unter dem Fuss durchgeführt, von der Innenseite her nach oben gezogen und auf der Beininnenseite durch einen Klettverschluss fixiert. So kann die Bandage in einer zweiten Phase als leichte Stütze verwendet werden.

Ein weiteres Ausführungsbeispiel für ein Gelenk, das anstelle von oder in Kombination mit jenem, das in den Figuren 4-6 dargestellt ist, eingesetzt werden kann, ist in den Figuren 9 und 10 dargestellt. Das Gelenk umfasst dabei zwei Bereiche: Der äussere Gelenkbereich 150 umfasst eine Gewindehülse 151, welche durch die äussere Gelenkfläche 152 mit dem Gelenkdeckel 153 fix verbunden ist und bei der Montage mit dem Gelenkdeckel 153 verpresst wird. Der innere Gelenkbereich 154 bildet sich aus einem Verdrehelement 155, das die innere Gelenkfläche 156 mit der Gegenplatte 157 verbindet. Die Verbindung zwischen Verdrehelement 155 und Gegenplatte 157 ist dabei eine Steckverbindung. Das Verdrehelement 155 besitzt neben dem Loch, durch das die Gelenkschraube 162 führt, noch eine 4-6-kant Vertiefung, welche mit einem Vorsprung auf der Gelenkplatte 157 kooperiert. So werden die beiden Teile zusammengesteckt und durch die Gelenkschraube 162 zusammengehalten, aber nicht fix verbunden wie im äusseren Gelenkbereich. Beide Bereiche 150 und 154 sind über ein Gewinde verbunden. Das Innengewinde 158 befindet sich dabei in der Gewindehülse, das passende Aussengewinde 159 auf dem Verdrehelement 155.

Die innere 156 und äussere Gelenkfläche 152 ist mit jeweils vier bogenförmigen Ausnehmungen oder Öffnungen 160 versehen, wobei die Öffnungen 160 jeweils um 90° um die Achse des Gelenks verschoben sind, so dass sich zwei Mal paarweise zwei gleiche Öffnungen gegenüberliegen. Auf der Gegenplatte 157 sind zwei gegenüberliegende Stifte 161 angeordnet, welche vom Fuss weg zeigen und in die bogenförmigen Öffnungen 160 auf der inneren 156 und äusseren Gelenkfläche 152 greifen. Auf der äusseren Gelenkfläche 152 besitzen die bogenförmigen Öffnungen 160a alle dieselbe Dimension und entsprechen in der Grösse den zwei gegenüberliegenden Stiften 161 auf der Gegenplatte 157. Die Öffnungen auf der inneren Gelenkfläche 156 verfügen hingegen über eine grössere Länge (in Umfangsrichtung) als die Stifte 161 auf der Gegenplatte 157. Konkret sind die gegenüberliegenden, bogenförmigen Öffnungen 160b (in Umfangsrichtung) länger als die gegenüberliegenden bogenförmigen Öffnungen 160c. Greifen die Stifte in die bogenförmigen Öffnungen 160b ist damit die Beweglichkeit um das Gelenk grösser. Wird die Gegenplatte 157 um 90° gedreht, greifen die Stifte in die bogenförmigen Öffnungen 160c und verringern so den Beweglichkeitsbereich des Gelenks. Je nachdem wie die Gegenplatte 157 positioniert wird, ist so eine Anpassung des Öffnungswinkels möglich und verschiedene Endanschläge für den Bewegungsspielraum sind je nach Therapiephase realisierbar.

Um das Anziehen der Schuhe zu erleichtern, lässt sich das ganze Stützelement 16 abklappen. Durch Drehung am Gelenkdeckel 153 und die fixe Verbindung mit der Gewindehülse 151 wird diese aus dem Verdrehelement 155 gedreht und schiebt so den äusseren 152 und inneren Gelenkbereich 156 auseinander. Dadurch greifen die Stifte 161 nicht mehr in die Ausnehmungen 160a der äusseren Gelenkfläche 152 und der gesamte äussere Gelenkbereich 150 ist entkoppelt. Das Gelenk lässt sich frei drehen und das Stützelement 16 kann bei geöffneten Bändern nach hinten abgeklappt werden, um den Einstieg zu erleichtern.

Damit der äussere Gelenkbereich 150 sich nicht komplett vom inneren Bereich 154 löst, sind beide Bereiche durch eine Gelenkschraube 162 verbunden, welche von aussen durch alle Gelenk-Elemente führt und dann in der Gegenplatte 157 verschraubt wird. Nach Entfernen der Gelenkschraube 162 lässt sich die Gegenplatte 157 wenigstens teilweise axial herausziehen, um die Gelenksachse drehen und die Positionierung der Stifte 161 bestimmen (Eingriff in Lochpaar 160b oder in Lochpaar 160c). In einer späteren Therapiephase ist zudem das gesamte Stützelement 16 mit dem äusseren Gelenkbereich 150 entfernbar.

**BEZUGSZEICHENLISTE**

| | | | |
|---|---|---|---|
| 11 | Laufsohle | 36 | Polsterung von 16 |
| 12 | Schaft | 37 | Spickel |
| 13 | Exoskelett | 38 | Klapplasche |
| 14 | äusseres Schalenelement | 39 | Klapplasche |
| 14a | nach oben aufkragende Erweiterung von 14 | 40 | Zwischenlasche mit Kanal |
| | | 41 | geteilte Zunge |
| 15 | inneres Schalenelement | 42 | Quartierteil |
| 15a | nach oben aufkragende Erweiterung von 15 | 43 | Quartierteil |
| | | 44 | Klettverschluss zur Befestigung von 39 an 38 |
| 16 | Stützelement | | |
| 17 | Manschette | 45 | elastisches Band |
| 18 | fussaussenseitige Versteifung | 46 | Klickverschluss |
| 19 | fussinnenseitige Versteifung | 47 | weiblicher Teil von 46 |
| 20 | äusseres Drehgelenk | 48 | männlicher Teil von 46 |
| 21 | inneres Drehgelenk | 49 | Umlenkrollen |
| 22 | Verschlusselement | 50 | Umlenkrolle |
| 23 | Umlenkrolle | 51 | Riemen |
| 24 | Ankerpunkt für 25 | 52 | Klettverschluss |
| 25 | vorderes dehnungsarmes Textilband | 53 | Einschnitt aussen |
| | | 54 | Einschnitt innen |
| 26 | Anbindungsfeld von 25 | 55 | Schnürsenkel |
| 27 | hinteres dehnungsarmes Textilband | 56 | Öse |
| | | 57 | Bremshaken |
| 28 | Fersenkappe | 58 | Haken |
| 29 | äussere Gelenkfläche | 59 | Stifte |
| 30 | innere Gelenkfläche | 59a | Breitere Stifte |
| 31 | PTFE-Scheibe in Gelenk | 59b | Stifte mit Verjüngung |
| 32 | Gegenplatte | 60 | bogenförmige Ausnehmungen |
| 33 | Innengewindeelement | | |
| 34 | Gelenkschraube | 61 | Riemen |
| 35 | Gelenksdeckel | 62 | Schlitz in 17 für 27 |
| 101 | Bandage | 131 | Umlenkung für 128 aussen |
| 102 | Exoskelett von 101 | 132 | Riemen |
| 103 | Stützelement | 133 | Anbindungsfeld für 128 |
| 104 | Schalenelement | 140 | innere Textilkletten |
| 105 | Fussschale | 141 | äussere Textilkletten |
| 106 | Manschette | 142 | äusseres dehnungsarmes textiles Kreuzband |
| 107 | fussinnenseitige Versteifung | | |
| 108 | fussaussenseitige Versteifung | 143 | inneres dehnungsarmes |
| 109 | inneres Drehgelenk | | textiles Kreuzband |
| 110 | äusseres Drehgelenk | 144 | elastisches Band |
| 111 | Verschlusselement | 145 | vorderes dehnungsarmes |
| 112 | Umlenkrolle | | Textilband |
| 113 | Polsterung von 103 | 150 | äusserer Gelenkbereich |
| 114 | Polsterung von 101 | 151 | Gewindehülse |
| 115 | hinteres dehnungsarmes Textilband | 152 | äussere Gelenkfläche |
| | | 153 | Gelenkdeckel |
| 116 | Schlitz in 106 für 115 | 154 | innerer Gelenkbereich |
| 117 | Druckknopf | 155 | Verdrehelement |
| 118 | inneres klappbares Seitenteil | 156 | innere Gelenkfläche |
| 119 | äusseres klappbares Seitenteil | 157 | Gegenplatte |
| 120 | geteilte Zunge | 158 | Innengewinde |
| 121 | Zwischenlasche | 159 | Aussengewinde |
| 122 | Schnürsenkel | 160 | bogenförmige Öffnungen |
| 123 | Ösen | 160a | bogenförmige Öffnung Grösse passend zu Stift 161 |
| 124 | Kanal auf 121 für 122 | | |
| 125 | Haken | 160b | bogenförmige Öffnungen grösser als 160c |
| 126 | Einschnitt innen | | |
| 127 | Einschnitt aussen | 160c | bogenförmige Öffnungen grösser als 160a |
| 128 | vorderes dehnungsarmes Textilband | | |
| | | 161 | Stifte |
| 129 | Umlenkung für 128 innen | 162 | Gelenkschraube |
| 130 | Riemen | | |

## Patentansprüche

1. Schuhwerk, oder bewegliche Fussstütze in Form einer Bandage, mit einer den Fuss wenigstens im Fersen- und hinteren Sohlenbereich, im Knöchelbereich und oberhalb des Knöchelbereichs umgreifenden flexiblen Schaftstruktur (12, 101) und einem wenigstens teilweise ausserhalb davon angeordneten Exoskelett (13, 102), das an und/oder in der Schaftstruktur (12, 101) befestigt ist, wobei das Exoskelett (13, 102)
ein in einem Sohlenbereich (11, 105) der Schaftstruktur (12, 101) befestigtes steifes Schalenelement (14,15,104) aufweist, das die Unterseite des Fusses wenigstens teilweise untergreift und das sich beidseits des Fusses in Form von Erweiterungen (14a, 15a) bis auf oberhalb der Höhe des Drehpunktes des Fussgelenks erstreckt,
sowie ein steifes Stützelement (16, 103), welches eine den Unterschenkel wenigstens teilweise oberhalb des Fussgelenks umlaufende Manschette (17, 106) aufweist, sowie zwei seitliche, nach unten gerichtete Versteifungen (18, 19), welche sich beidseits des Fusses bis auf unterhalb der Höhe des Drehpunktes des Fussgelenks erstrecken,
wobei Erweiterungen (14a, 15a) und Versteifungen (18,19) beidseits des Fusses über je ein fussaussenseitiges und ein fussinnenseitiges Gelenk (20,21,109,110) verschwenkbar verbunden sind,
und wobei der maximale Öffnungswinkel des Gelenks (20, 21,109,110) bezüglich Plantarflexion begrenzt ist durch wenigstens ein dehnungsarmes Band (25,128, 142,143), welches wenigstens eine Erweiterung (14a, 15a) fussspitzenseitig mit einem schienbeinseitigen oder seitlichen Bereich der Manschette (17,106) einstellbar verbindet.

2. Schuhwerk oder Fussstütze nach Anspruch 1, **dadurch gekennzeichnet, dass** das Band (25,128,143) an der fussaussenseitigen Erweiterung (14a) im Bereich deren der Fussspitze zugewandten Kante befestigt ist und über den Talus laufend am fussinnenseitigen Bereich der Manschette (17,107) einstellbar festgelegt werden kann, vorzugweise über eine Klettverbindung (26) oder einen Druckknopfverschluss
und/oder dass das Band (142) an der fussinnenseitigen Erweiterung (15a) im Bereich deren der Fussspitze zugewandten Kante befestigt ist und über den Talus laufend am fussaussenseitigen Bereich der Manschette (17,107) einstellbar festgelegt werden kann, vorzugweise über eine Klettverbindung (26) oder einen Druckknopfverschluss.

3. Schuhwerk oder Fussstütze nach einem der vorhergehenden Ansprüche, **dadurch gekennzeichnet, dass** zwischen Sohlenbereich (11, 105) und fersenseitiger Manschette ein dehnungsarmes einstellbares zweites Band (27,115) angeordnet ist, vorzugsweise indem es im Sohlenbereich befestigt, durch einen horizontalen Schlitz (129,116) in der Manschette (17,106) geführt und nach unten umgelegt ist und variabel festgelegt werden kann, vorzugsweise über eine Klettverbindung oder einen Druckknopfverschluss (117).

4. Schuhwerk oder Fussstütze nach einem der vorhergehenden Ansprüche, **dadurch gekennzeichnet, dass** es im den Knöchel umgreifenden Bereich sowie über wenigstens 50 %, insbesondere bevorzugt wenigstens 70 % der Ristlänge öffenbar ist.

5. Schuhwerk oder Fussstütze nach Anspruch 4, **dadurch gekennzeichnet, dass** im den knöchelumgreifenden Bereich eine zentral geteilte Zunge (41,120) angeordnet ist, welche über eine Schnürung und/oder einen Klettverschluss verschlossen werden kann.

6. Schuhwerk oder Fussstütze nach einem der vorhergehenden Ansprüche 4 oder 5, **dadurch gekennzeichnet, dass** im Übergangsbereich zwischen dem knöchelumgreifenden Bereich und dem Ristbereich eine dahinterliegende Zwischenlasche (40,121) angeordnet ist.

7. Schuhwerk oder Fussstütze nach einem der vorhergehenden Ansprüche 4, 5 oder 6, **dadurch gekennzeichnet, dass** im Ristbereich die Einstiegsöffnung für den Fuss über wenigstens eine Klapplasche (38, 39, 118, 119) verschliessbar ist, vorzugsweise über 2 von der Fussinnenseite respektive der Fussaussenseite zur Mitte zum Rist und wenigstens Bereichsweise übereinandergelegt verschliessbare Klapplaschen (38, 39, 118, 119).

8. Schuhwerk oder Fussstütze nach Anspruch 7, **dadurch gekennzeichnet, dass** die fussaussenseitige Klapplasche (38, 119) über ein elastisches Band (45, 128) verfügt, welches insbesondere bevorzugt auf der Fussinnenseite, vorzugsweise an der inneren Erweiterung (15a), festgelegt oder wieder nach aussen umgelegt und nach aussen festgelegt werden kann.

9. Schuhwerk oder Fussstütze nach einem der vorhergehenden Ansprüche, **dadurch gekennzeichnet, dass** das Schalenelement (14, 15, 104) einstückig U-förmig ausgebildet ist oder zwei getrennte L-förmige Elemente (14,15) aufweist, ein äusseres (14) und ein inneres (15), wobei vorzugsweise die äussere Erweiterung (14a) sich weiter zur Fussspitze erstreckt als die innere (15a).

10. Schuhwerk oder Fussstütze nach einem der vorhergehenden Ansprüche, **dadurch gekennzeichnet, dass** die Gelenke (20, 21, 109, 110) lösbar ausgestaltet sind, sodass das Stützelement (16, 103) im fortgeschrittenen Therapiestadium entfernt werden kann, wobei vorzugsweise die Gelenke (20, 21, 109, 110) über eine innere, dem Fuss zugewandte Gegenplatte (32, 156) verfügen, welche eine nach aussen gerichtete, mit einem Innengewinde versehene hohlzylindrische Erweiterung (33) aufweisen, deren Aussenfläche vorzugsweise die Drehachse bildet, und das Gelenk (20, 21, 109, 110) durch eine von aussen eingeschraubte Kopfschraube (34,162) mit in das Innengewinde der hohlzylindrischen Erweiterung (33) eingreifendem Aussengewinde zusammengehalten ist, und wobei weiterhin vorzugsweise zwischen aneinander gleitenden Elementen des jeweiligen Gelenks Gleitelemente, insbesondere vorzugsweise PTFE-Scheiben (31), angeordnet sind.

11. Schuhwerk oder Fussstütze nach einem der vorhergehenden Ansprüche, **dadurch gekennzeichnet, dass** die Manschette (17, 106) als zur Fussspitze eine Einstiegsausnehmung aufweisende Ringmanschette ausgebildet ist, deren Einstiegsausnehmung vorzugsweise über ein bandförmiges, vorzugsweise dehnungsarmes Verschlusselement (22, 111) geschlossen werden kann, insbesondere bevorzugt über eine Umlenkrolle auf sich selber zurückgelegt über einen Klettverschluss.

12. Schuhwerk oder Fussstütze nach einem der vorhergehenden Ansprüche, **dadurch gekennzeichnet, dass** das fussinnenseitige und/oder das fussaussenseitige Gelenk (20, 21) mit Endanschlägen für die Plantarflexion und/oder Dorsalextension versehen ist, wobei diese Endanschläge vorzugsweise durch mindestens zwei bezüglich der Gelenksachse gegenüberliegende, bogenförmige Öffnungen oder Ausnehmungen (60,160), vorzugsweise in den Elementen der Versteifungen (18, 19), realisiert sind, in welche zwei bezüglich des anderen Gelenkelements (14a, 15a) ortsfeste Stifte (59, 161), die vorzugsweise an einem bezüglich des anderen Gelenkelements (14a, 15a) fixierten Gelenkdeckel (35,157) parallel zur Gelenkachse angeordnet sind, eingreifen, wobei insbesondere bevorzugt am Gelenkdeckel (35) auf zwei bezüglich Deckelebene gegenüberliegenden Seiten Stifte (59) mit unterschiedlicher in die Öffnungen oder Ausnehmungen (60) eingreifender Länge in Umlaufrichtung, angeordnet sind, oder am Gelenkdeckel (157) ein Paar von zwei bezüglich Gelenkachse gegenüberliegenden Stiften (161) mit jeweils gleicher Länge in Umlaufrichtung in die Öffnungen oder Ausnehmungen (160) unterschiedlicher Länge eingreifend, angeordnet sind.

13. Schuhwerk oder Fussstütze nach Anspruch 12, **dadurch gekennzeichnet, dass** die Endanschläge so ausgelegt sind, dass sie erst zum Anschlag kommen, wenn die Beweglichkeit um das Gelenk durch das wenigstens eine dehnungsarme Band (25, 128) respektive das dehnungsarme einstellbare zweite Band (27, 115) begrenzt ist und das Gelenk über diese Begrenzung hinaus belastet wird.

14. Schuhwerk nach einem der vorhergehenden Ansprüche, **dadurch gekennzeichnet, dass** die Schaftstruktur (12) als geschlossener Schuh mit Sohle ausgebildet ist.

15. Bewegliche Fussstütze in Form einer Bandage nach einem der vorhergehenden Ansprüche, **dadurch gekennzeichnet, dass** die Bandage eine Fussschale (105) aufweist, welche auf der Unterseite des Fusses einschliesslich des Fersenbereichs aber vorzugsweise ohne Zehenbereich angeordnet ist, welche sich über die gesamte Breite des Fusses erstreckt, und an welcher das Schalenelement (104) an geformt oder befestigt ist.
